# EUROPÄISCHE PATENTSCHRIFT

(11) **EP 2 664 303 B1**
(45) Veröffentlichungstag und Bekanntmachung des Hinweises auf die Patenterteilung: **04.03.2015**
(21) Anmeldenummer: 13002061.3
(22) Anmeldetag: 19.04.2013
(51) Int. Cl.: A61F 2/60

(54) **Prothese mit einem Prothesenschaft**
Prosthesis comprising a prosthesis shaft
Prothèse dotée d'une tige de prothèse

(30) Priorität: 15.05.2012 DE 102012009701
(43) Veröffentlichungstag der Anmeldung: 20.11.2013
(73) Patentinhaber: Otto Bock HealthCare GmbH, 37115 Duderstadt (DE)
(72) Erfinder: Hillmann, Martin, 37115 Duderstadt (DE)
(74) Vertreter: Lins, Edgar

(56) Entgegenhaltungen:
- WO-A1-03/041619
- DD-A1- 135 800
- DE-U1-202004 007 308
- US-A- 6 013 105
- US-B1- 6 440 173

## Beschreibung

Die Erfindung betrifft eine Prothese mit einem zur Aufnahme eines Amputationsstumpfs ausgebildeten Prothesenschaft, der an seinem distalen Ende eine Befestigungseinrichtung zur Halterung eines künstlichen Glieds aufweist, wobei die Befestigungseinrichtung ein flächiges Basisteil mit einem ringförmigen Rand und ein um eine senkrecht zum flächigen Basisteil stehende Längsachse drehbar relativ zum Basisteil positionierbares und der Befestigung des künstlichen Glieds dienendes Befestigungsmittel aufweist, das in einer eingestellten Winkelposition relativ zum Basisteil mittels einer Fixiereinrichtung feststellbar ist und wobei der ringförmige Rand einen kreisförmigen Umfang des Befestigungsmittels führend umgibt.

Die übliche Befestigung eines künstlichen Gliedes besteht darin, dass ein Prothesenschaft das Restglied bzw. den Amputationsstumpf des amputierten Gliedes aufnimmt und so die Verbindung mit dem die amputierte Gliedmaße ersetzenden künstlichen Glied herstellt. Bei einer Beinprothese kann der Prothesenschaft einen Unterschenkel-Amputationsstumpf oder einen Oberschenkel-Amputationsstumpf umfassen. Ein Prothesenschaft für einen Unterschenkel-Amputationsstumpf umfasst beispielsweise ein Residuum des Unterschenkels und einen künstlichen Fuß, während sich bei einem Oberschenkel-Amputationsstumpf an den Prothesenschaft zusätzlich ein künstliches Kniegelenk anschließen kann.

Es ist üblich geworden, den Prothesenschaft nicht unmittelbar auf den Amputationsstumpf aufzubringen, sondern den Amputationsstumpf mit einem polsternden und schützenden Liner zu versehen, bevor er in den proximal offenen und distal geschlossenen Prothesenschaft eingeführt wird. Dabei kann vorgesehen sein, dass am distalen Ende des Liners ein Verriegelungsbolzen angebracht ist, der durch eine Durchgangsöffnung am distalen Ende des Prothesenschafts hindurchreicht und in eine Schlossanordnung einführbar ist, die an der distalen Unterseite des Prothesenschafts befestigt und das Einführen des Verriegelungsbolzens nach distal ermöglicht, eine Rückzugsbewegung nach proximal jedoch sperrt. Auf diese Weise wird der mit dem Liner versehene Amputationsstumpf in dem Prothesenschaft in einer maximal distalen Anschlagstellung verriegelt. Das Herausziehen des Amputationsstumpfs aus dem Prothesenschaft erfordert eine Entriegelung der Schlossanordnung mittels eines Entriegelungsknopfes oder einer Entriegelungstaste.

Es hat sich herausgestellt, dass unterschiedliche Patienten eine unterschiedliche Positionierung des Entriegelungsknopfes oder der Entriegelungstaste bevorzugen oder eine geeignete Positionierung erst in Versuchen ermitteln müssen. Da die Schlossanordnung fest mit dem Prothesenschaft verbunden ist, ergeben sich jeweils Probleme für eine neue Anordnung der Schlossanordnung.

In vielen Fällen muss das künstliche Glied bezüglich der Längsachse des Prothesenschafts (von proximal nach distal) bezüglich seiner Winkelstellung justiert werden. Diese Justierung wird an dem künstlichen Glied dadurch vorgenommen, dass ein Justieradapter vorgesehen ist, der mit einer kugelkalottenförmigen Endung eines Teilelements des künstlichen Glieds zusammenwirkt und eine Justierung der Winkelstellungen um mehrere Raumachsen ermöglicht. Wird eine Justierung nur um eine Achse gewünscht, muss sorgfältig darauf geachtet werden, dass nicht zugleich eine Dejustierung bezüglich einer senkrecht dazu stehenden Drehachse erfolgt.

Eine Prothese der eingangs erwähnten Art ist durch US 6 013 105 bekannt. Der Prothesenschaft ist dabei an seinem distalen Ende mit einer Verdickung versehen, in die Gewinde für die Aufnahme von Befestigungsschrauben einsetzbar sind. Mit den Befestigungsschrauben ist eine Befestigungseinrichtung an der Außenseite des distalen Endes des Prothesenschafts anschraubbar. Die Befestigungseinrichtung weist einen kreiszylindrischen, topfförmigen Basiskörper auf, in den ein Befestigungsteil mit einem zylindrischen Außendurchmesser einsetzbar ist. Das Befestigungsteil weist eine distale Schulter auf, über die ein Innenflansch eines Befestigungsrings ragt. Der Befestigungsring weist ein Innengewinde auf, das mit einem Außengewinde des Basiskörpers zusammenwirkt und so beim Aufschrauben das Befestigungsteil in dem Basiskörper festklemmen kann. Durch Lockern des Befestigungsrings lässt sich das Befestigungsteil in dem Basisteil verdrehen und durch erneutes Anziehen des Befestigungsrings in der gedrehten Lage fixieren. Mit dem Befestigungsteil ist ein Verbindungsadapter zu einem Prothesenelement verbunden. Durch das Aufschrauben der Befestigungseinrichtung an der Außenseite des distalen Endes des Adapters wird ein relativ großvolumiger Aufbau benötigt.

Eine ähnliche Lösung ist durch DD 135 800 bekannt. Die eine Drehung ermöglichende Verstelleinrichtung ist als Adapterteil ausgebildet. Durch schräge Gleitflächen wird durch die Drehbewegung um eine vertikale Achse eine Winkelstellung in einer vertikalen Ebene, die die vertikale Achse einschließt, veränderbar.

DE 20 2004 007 308 U1 offenbart eine Unterschenkelprothese, bei der ein mit einem künstlichen Fuß verbundener Rohransatz in einer Rohrschelle drehbar angeordnet und durch eine Fixiereinrichtung in Form einer Rohrschelle fixierbar ist. Eine Ausbildung eines Prothesenschafts ist nicht offenbart.

Der vorliegenden Erfindung liegt die Aufgabe zugrunde, eine Prothese, insbesondere Beinprothese, der eingangs erwähnten Art hinsichtlich der Einstellmöglichkeiten zu verbessern.

Zur Lösung dieser Aufgabe ist erfindungsgemäß eine Prothese der eingangs erwähnten Art dadurch gekennzeichnet, dass das Basisteil mit seinem ringförmigen Rand drehfest in das distale Ende des Prothesenschafts eingeformt ist und dass die Fixiereinrichtung zur Festellung der Winkelposition aufgrund einer mittels wenigstens einer von der distalen Unterseite des Prothesenschafts betätigbaren Schraubverbindung ausgeübten Axialkraft ausgebildet ist.

Erfindungsgemäß ist somit eine Verstellmöglichkeit der Prothese bereits am distalen Ende des Prothesenschafts vorgesehen. Die dadurch mögliche Verstellung ist eine Winkelverstellung um eine Längsachse, die sich von proximal nach distal erstreckt und senkrecht zur Befestigungsfläche des flächigen Basisteils steht. Das Basisteil ist drehfest in den Schaft eingeformt, bevorzugt bei der Herstellung des Schafts. Das Befestigungsmittel ist in der eingestellten Winkelposition relativ zum Basisteil mittels einer Fixiereinrichtung feststellbar, wobei die Fixiereinrichtung von der distalen Unterseite des Prothesenschafts aus betätigbar ist.

Das relativ zum Basisteil in seiner Winkelposition einstellbare Befestigungsmittel lässt sich bei der Drehverstellung vereinfacht handhaben, wenn das Basisteil mit einer Führung für das relativ zum Basisteil drehbare Befestigungsmittel versehen ist. Hierfür weist das Basisteil ringförmigen Rand auf, der einen kreisförmigen Umfang des Befestigungsmittels führend umgibt.

Die Fixiereinrichtung ist zur Feststellung der Winkelposition aufgrund einer ausgeübten Axialkraft ausgebildet. Hierfür ist es beispielsweise denkbar, dass das Befestigungsmittel und das Basisteil jeweils zueinander zeigende komplementär geriffelte oder sonst wie strukturierte Oberflächen aufweisen, die nach Lösen einer Axialkraft voneinander entfernbar und zueinander drehbar sind und durch Anziehen der Axialkraft in eine verriegelte Position gelangen. In einer Variante könnte das Befestigungsmittel eine kreisförmige Umfangslinie mit einer Außenverzahnung aufweisen, die in eine komplementäre Innenverzahnung des Rands des Basisteils eingreifen kann. Durch Lösen der Axialkraft könnte das Befestigungsmittel aus dem Bereich des Randes des Basisteils herausgehoben und in seiner Winkelstellung verändert werden, um dann durch erneutes Anziehen der Axialkraft mittels der komplementären Verzahnung am Außenrand des kreisförmigen Umfangs des Befestigungsmittels fixiert zu werden.

>

In einer bevorzugten Ausführungsform der Erfindung weist das Befestigungsmittel eine gegen einen ersten axialen Anschlag des Basisteils mit einer Axialkraft ziehbare Befestigungsplatte und eine die Axialkraft ermöglichende, sich am Basisteil axial abstützende Gegenplatte auf. Die Gegenplatte kann sich dabei am Basisteil abstützen oder fest mit dem Basisteil verbunden oder einstückig mit dem Basisteil ausgebildet sein.

In einer einfachen und mit Vorteil einzusetzenden Ausführungsform ist das Basisteil als ein Ring mit einer den axialen Anschlag für das Befestigungsmittel aufweisenden Innenwandung ausgebildet. Ist die Gegenplatte ein separates Teil, weist die Innenwandung in einer bevorzugten Ausführungsform axiale Anschläge in beiden axialen Richtungen auf, sodass sich die Gegenplatte an einem der axialen Anschläge in bezüglich der Befestigungsplatte entgegengerichteten Richtung abstützt. Die beiden Anschläge können dabei an einem gemeinsamen, nach radial innen ragenden Steg der Innenwand des ringförmigen Basisteils ausgebildet sein.

Es ist vorteilhaft, wenn die separate Gegenplatte ebenfalls einen runden Außenumfang aufweist und ein ringförmiger Rand des Basisteils die Gegenplatte führend umgibt. Die Fixierung der Befestigungsplatte relativ zum Basisteil mittels der Gegenplatte erfolgt vorzugsweise durch eine Klemmung, für die die Axialkraft mit unterschiedlichen Mechanismen aufgebracht werden kann. In einer konstruktiv einfachen Ausführungsform wird die Axialkraft mittels wenigstens einer Schraubverbindung aufgebracht, wobei in der Gegenplatte ein Gewindeloch für einen Schraubbolzen vorgesehen sein kann, der mit seinem Kopf von der distalen Unterseite des Prothesenschafts betätigbar ist.

Bei der Ausführungsform des Befestigungsmittels mit einer Befestigungsplatte und einer Gegenplatte kann die Befestigungsplatte, aber auch die Gegenplatte zusammen mit oder auch ohne Befestigungsplatte das künstliche Glied am Prothesenschaft befestigen. Eine elegante Lösung besteht darin, dass ein Ansatz des künstlichen Glieds mit einem Außengewinde und einem nach radial außen vorstehenden Anschlag ausgebildet und in ein durch die Befestigungsplatte und/oder die Gegenplatte gebildetes Innengewinde bis zu dem Anschlag einschraubbar ist. In dieser Ausführungsform können Befestigungsplatte und Gegenplatte unmittelbar aneinander liegen und mit sich gegenseitig vorsetzenden Innengewindeabschnitten versehen sein, sodass die Befestigung des künstlichen Gliedes gemeinsam durch Befestigungsplatte und Gegenplatte erfolgt. Durch das axiale Einschrauben des Ansatzes des künstlichen Gliedes bis zum Anschlag, der dann an der Befestigungsplatte anliegt, wird eine gegenüber bisherigen Befestigungsmöglichkeiten verbesserte und dauerhaltbare Verbindung hergestellt.

Der an der Unterseite des distalen Endes des Prothesenschafts befestigte Teil des künstlichen Gliedes kann auch eine mit dem künstlichen Glied verbundene Schlossanordnung zur Aufnahme eines Befestigungsstifts des Liners sein. Dessen Entriegelungsknopf oder -taste kann aufgrund der erfindungsgemäßen Anordnung in jeder radialen Winkelposition ohne Schwierigkeiten angeordnet werden, ohne eine etwaige vorherige Justierung des künstlichen Gliedes zu gefährden.

Die Erfindung soll im Folgenden anhand eines in der Zeichnung dargestellten Ausführungsbeispiels näher erläutert werden. Es zeigen
- Figur 1 -: eine Seitenansicht einer Unterschenkelprothese mit einem Prothesenschaft und eines am distalen Ende des Prothesenschafts gehaltenen künstlichen Glieds,
- Figur 2 -: einen Schnitt durch die Anordnung gemäß Figur 1, gesehen von vorn,
- Figur 3 -: eine vergrößerte Teildarstellung des Prothesenschafts und seiner Verbindung zum künstlichen Glied gemäß Figur 2,
- Figur 4 -: eine explodierte Darstellung der gemäß Figur 3 verwendeten Befestigungseinrichtung.

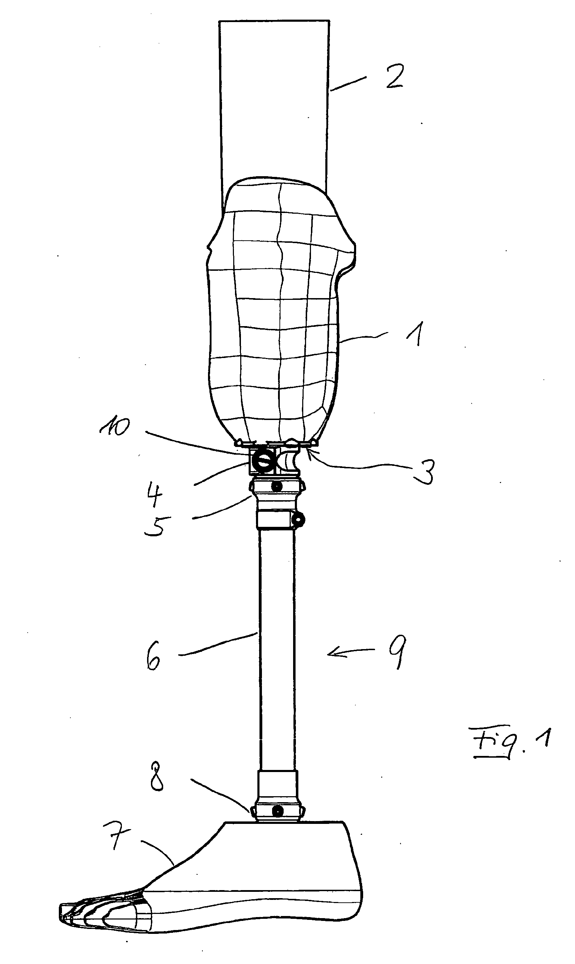

Figur 1 lässt einen Prothesenschaft 1 erkennen, der üblicherweise aus einem duroplastischen Kunststoff hergestellt und an die Form des (nicht dargestellten) Amputationsstumpfs des Patienten angepasst ist. In den Prothesenschaft 1 ist schematisch ein zylindrischer Liner 2 eingesetzt, der jedoch üblicherweise zunächst auf den Amputationsstumpf aufgezogen, beispielsweise aufgerollt wird, bevor der Amputationsstumpf in den Prothesenschaft eingeführt wird. An der distalen Unterseite des Prothesenschafts 1 befindet sich eine Befestigungseinrichtung 3, mit der eine Schlosseinrichtung 4 an dem Prothesenschaft befestigt ist. Die Schlosseinrichtung 4 ist über eine Justierverbindung 5 mit einem Unterschenkel-Modulrohr 6 verbunden, an das sich an seinem distalen Ende ein künstlicher Fuß 7 über eine weitere Justierverbindung 8 anschließt.

Die Schlosseinrichtung 4, die Justierverbindungen 5, 8, das Unterschenkel-Modulrohr 6 und der künstliche Fuß 7 bilden ein am Prothesenschaft 1 befestigtes künstliches Glied 9, das mittels des Prothesenschafts 1 und ggf. dem Liner 2 am Amputationsstumpf befestigt wird. Die Schlosseinrichtung 4 weist eine Entriegelungstaste 10 auf, die betätigt werden muss, wenn der Amputationsstumpf mit dem Liner 2 aus dem Prothesenschaft 1 zum Ablegen der Prothese herausgezogen werden soll.

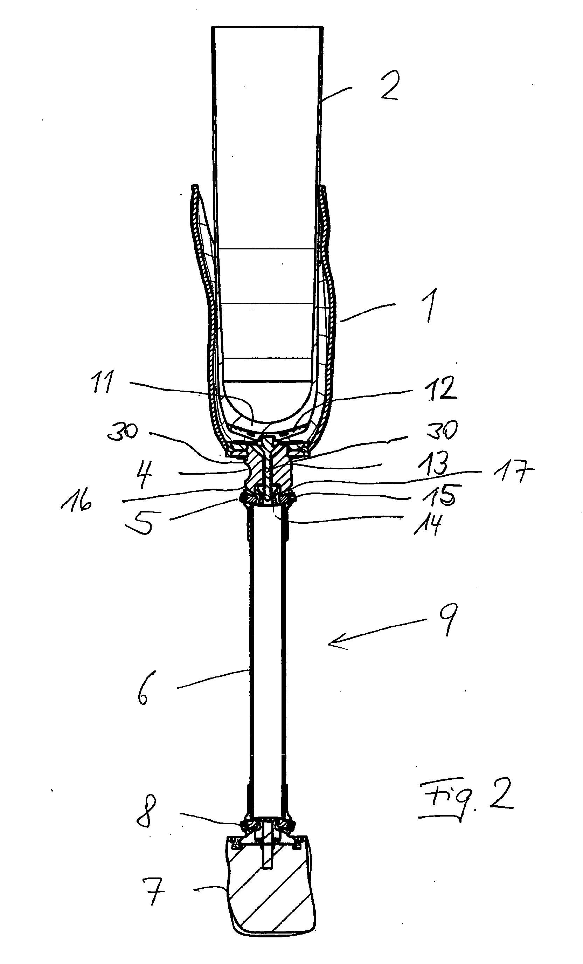

Die Schnittdarstellung in Figur 2 verdeutlicht, dass der Liner 2 im Wesentlichen zylindrisch ausgebildet ist und ein geschlossenes distales Ende 11 aufweist. Am distalen Ende 11 weist der Liner 2 eine größere Wandstärke auf, sodass er dort eine verstärkte Polsterwirkung ausübt. In das distale Ende 11 ist eine Schirmstruktur 12 eingeformt, die einen Verriegelungsbolzen 13 trägt. Der Verriegelungsbolzen 13 ragt in einen proximal trichterförmig erweiterten Kanal der Schlossanordnung 4. Der Verriegelungsbolzen 13 weist an einer Oberfläche ringförmige parallele Stege auf, die beispielsweise mit einer entsprechenden Zahnradstruktur der Schlosseinrichtung 4 in bekannter Weise zusammenwirken, wobei die Zahnradstruktur eine Rotation nur in einer Richtung ermöglicht, während die Rotation in der anderen Richtung durch ein Gesperre verhindert wird. In der Praxis bedeutet dies, dass der Verriegelungsbolzen durch das Gewicht des Patienten relativ zum Prothesenschaft 1 nach unten (distal) gedrückt werden kann, das Herausziehen des Verriegelungsbolzens 13 aus der Schlosseinrichtung 4 jedoch verhindert wird, solange nicht die Entriegelungstaste 10 gedrückt wird.

Figur 2 verdeutlicht, dass die Justierverbindung 5 in bekannter Weise aus Schrägflächen 14 einer umgekehrten Pyramide besteht, deren Position relativ zu dem Modulrohr 6 mittels senkrecht zu den Schrägflächen 14 verlaufender Madenschrauben 15 fixiert wird. Die umgekehrte Pyramide ragt aus einem Kugelkalottensegment 16 heraus, das mit einer entsprechenden komplementären Gegenfläche 17 zur Justierung zusammenwirkt.

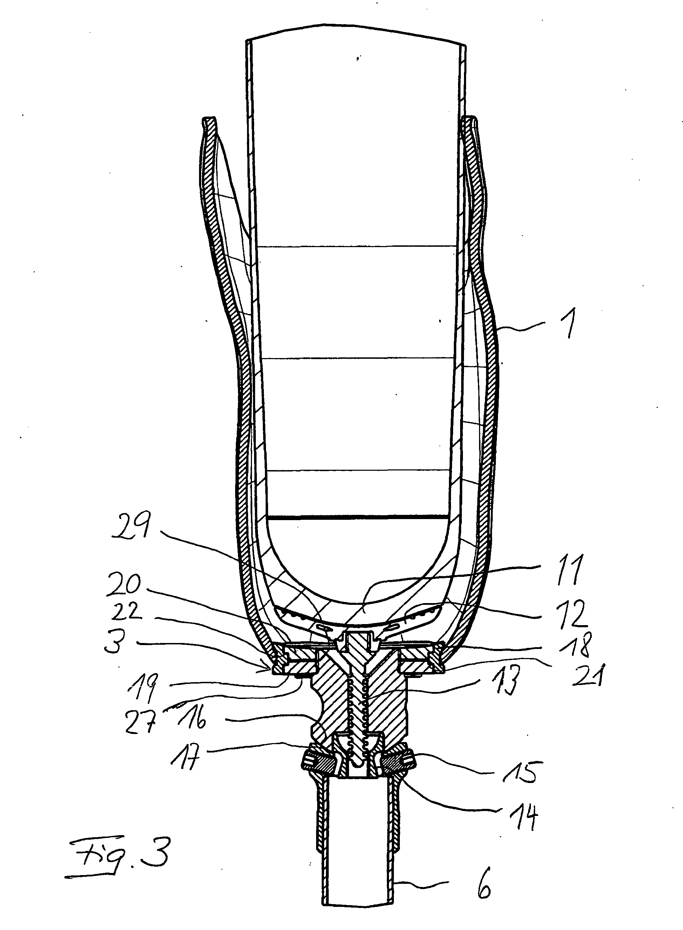

Die vergrößerte Darstellung in Figur 3 lässt erkennen, dass die Befestigungseinrichtung aus einem in das distale Ende des Prothesenschafts 1 eingeformten Basisteil 18 und einem in das Basisteil 18 eingesetzten Befestigungsmittel besteht, das eine Befestigungsplatte 19 und eine Gegenplatte 20 aufweist. Das Basisteil 18 ist in Form eines Rings ausgebildet, der auf seiner Innenwand einen mittigen umlaufenden Vorsprung aufweist, der einen axialen Anschlag 21 für die Befestigungsplatte 19 und einen entgegen gerichteten axialen Anschlag 22 für die Gegenplatte 20. Damit die Befestigungsplatte 19 und die Gegenplatte 20 im montierten Zustand aneinander anliegen können, sind sie mit einer den Anschlägen 21, 22 entsprechenden Abstufung versehen.

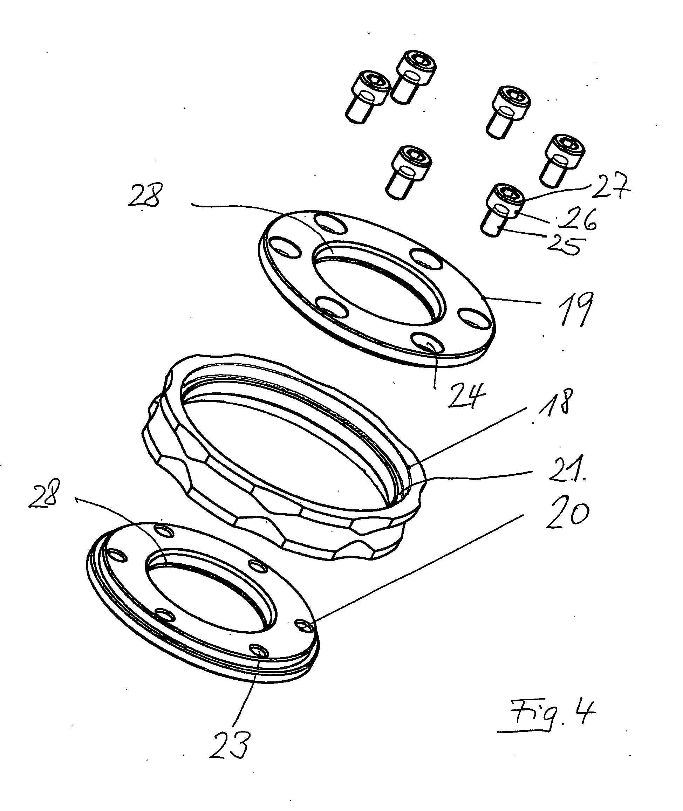

Wie Figur 4 verdeutlicht, weist die Gegenplatte 20 Gewindelöcher 23, vorzugsweise in Form von Sacklöchern, auf, die mit Durchgangsöffnungen 24 in der Befestigungsplatte 19 fluchten. Durch die Durchgangsöffnungen 24 ragen Gewindebolzen 25 von Schrauben 26, deren Schraubköpfe 27 in den bestuft ausgebildeten Ausgangsöffnungen 24 im montierten Zustand zumindest teilweise versenkt sind. Die Gewindebolzen 25 sind im montierten Zustand in die Gewindelöcher 23 eingeschraubt. Das Basisteil 18 lässt in Figur 4 den ringförmig an der Innenwandung umlaufenden axialen Anschlag 22 erkennen, wobei die Innenwandung des Basisteils 18 einen Innendurchmesser aufweist, der eine führende Aufnahme der Befestigungsplatte 19 und zumindest eines Teils der Gegenplatte 23 ermöglicht, sodass sich die montierte Struktur der Figur 3 ergibt.

Durch Lösen der eine axiale Klemmkraft ausübenden, als Fixiereinrichtung dienenden Schraubverbindung 23, 26 werden die Befestigungsplatte 19 und Gegenplatte 20 von den Anschlägen 21, 22 gelöst, sodass beide Platten in ihrem durch die gelockerten Schraubverbindungen 23, 26 verbundenen Zustand relativ zu dem Basisteil 18 um eine vertikale Längsachse gedreht werden können, um so die Winkelposition des künstlichen Glieds 9 relativ zum Prothesenschaft 1 - und damit relativ zum Amputationsstumpf - einstellen zu können. Hierfür ist das künstliche Glied 9 drehfest mit dem Befestigungsmittel verbunden. In dem dargestellten Ausführungsbeispiel liegen Befestigungsplatte 19 und Gegenplatte 20 im montierten und geklemmten Zustand dicht aneinander an und sind beide ringförmig ausgebildet, sodass sie auf ihrer radialen Innenfläche Innengewindeabschnitte 28 aufweisen, die aneinander anschließen. Ein zylindrischer Ansatz 29 des künstlichen Glieds 9 (hier der Schlossanordnung 4) ist somit mit einem Außengewinde in die Befestigungsplatte 19 und die Gegenplatte 20, die durch die Schraubverbindung 23, 26 miteinander verspannt sind, einschraubbar, und zwar bis zu einem durch eine stufenförmige Erweiterung im distalen Anschluss an den Ansatz 29 gebildeten ringförmigen Anschlag, sodass das künstliche Glied mit dem Ansatz 29 durch eine axiale Verschraubung fest verbunden ist.

Durch die Einstellung der Winkelposition ist es beispielsweise möglich, die Entriegelungstaste 10 in eine für den jeweiligen Patienten genehme Position zu bringen, also beispielsweise in eine laterale oder in eine eher nach frontal oder dorsal ausgerichtete Position einzustellen.

Unabhängig von dem Vorhandensein einer Schlosseinrichtung 4 kann durch die erfindungsgemäße Befestigung das künstliche Glied 9 bereits am distalen Ende des Prothesenschafts 1 in eine gewünschte Winkelposition bzgl. der Längsachse eingestellt werden.

## Patentansprüche

1. Prothese mit einem zur Aufnahme eines Amputationsstumpfs ausgebildeten Prothesenschaft (1), der an seinem distalen Ende eine Befestigungseinrichtung (3) zur Halterung eines künstlichen Glieds (9) aufweist, wobei die Befestigungseinrichtung ein flächiges Basisteil (18) mit einem ringförmigen Rand und ein um eine senkrecht zum flächigen Basisteil (18) stehende Längsachse drehbar relativ zum Basisteil (18) positionierbares und der Befestigung des künstlichen Glieds (9) dienendes Befestigungsmittel aufweist, das in einer eingestellten Winkelposition relativ zum Basisteil (18) mittels einer Fixiereinrichtung feststellbar ist und wobei der ringförmige Rand einen kreisförmigen Umfang des Befestigungsmittels führend umgibt, **dadurch gekennzeichnet, dass** das Basisteil (18) mit seinem ringförmigen Rand drehfest in das distale Ende des Prothesenschafts (1) eingeformt ist und dass die Fixiereinrichtung zur Festellung der Winkelposition aufgrund einer mittels wenigstens einer von der distalen Unterseite des Prothesenschafts (1) betätigbaren Schraubverbindung (23, 26) ausgeübten Axialkraft ausgebildet ist.

2. Prothese nach Anspruch 1, **dadurch gekennzeichnet, dass** das Befestigungsmittel eine gegen einen ersten axialen Anschlag (21) des Basisteils (18) mit einer Axialkraft ziehbare Befestigungsplatte (19) und eine die Axialkraft ermöglichenden, sich am Basisteil (18) axial abstützende Gegenplatte (20) aufweist.

3. Prothese nach Anspruch 2, **dadurch gekennzeichnet, dass** das Basisteil (18) als ein Ring mit einer den ersten axialen Anschlag (21) für das Befestigungsmittel aufweisenden Innenwandung ausgebildet ist.

4. Prothese nach Anspruch 3, **dadurch gekennzeichnet, dass** die Innenwandung axiale Anschläge (21, 22) in beide axiale Richtungen aufweist und dass sich die Gegenplatte (20) an einem der axialen Anschläge (21, 22) in bezüglich der Befestigungsplatte (19) entgegensetzter Richtung abstützt.

5. Prothese nach Anspruch 3 oder 4, **dadurch gekennzeichnet, dass** die Gegenplatte (20) einen runden Außenumfang aufweist und dass ein ringförmiger Rand des Basisteils (18) die Gegenplatte (20) führend umgibt.

6. Prothese nach Anspruch 2 oder 3, **dadurch gekennzeichnet, dass** die Gegenplatte (20) fest mit dem Basisteil (18) verbunden oder als Teil des Basisteils (18) ausgebildet ist.

## Claims

1. Prosthesis with a prosthesis socket (1) which is designed to receive an amputation stump and which, at its distal end, has a fastening device (3) for retaining an artificial limb (9), wherein the fastening device has a planar base part (18), with an annular edge, and a fastening means which can be positioned relative to the base part (18), by being rotatable about a longitudinal axis arranged perpendicular to the planar base part (18), and serves to fasten the artificial limb (9), said fastening means being able to be set in an adjusted angle position relative to the base part (18) by means of a fixing device, and wherein the annular edge surrounds and guides a circular circumference of the fastening means, **characterized in that** the annular edge of the base part (18) is formed in a rotationally fixed manner into the distal end of the prosthesis socket (1), and **in that** the fixing device is designed to set the angle position on the basis of an axial force exerted by means of at least one screw connection (23, 26) that can be actuated from the distal underside of the prosthesis socket (1).

2. Prosthesis according to Claim 1, **characterized in that** the fastening means has a fastening plate (19), which can be drawn with an axial force against a first axial abutment (21) of the base part (18), and a counter-plate (20), which permits the axial force and bears axially on the base part (18).

3. Prosthesis according to Claim 2, **characterized in that** the base part (18) is designed as a ring with an inner wall having the first axial abutment (21) for the fastening means.

4. Prosthesis according to Claim 3, **characterized in that** the inner wall has axial abutments (21, 22) in both axial directions, and **in that** the counter-plate (20) bears on one of the axial abutments (21, 22) in an opposite direction with respect to the fastening plate (19).

5. Prosthesis according to Claim 3 or 4, **characterized in that** the counter-plate (20) has a round outer circumference, and **in that** an annular edge of the base part (18) surrounds and guides the counter-plate (20).

6. Prosthesis according to Claim 2 or 3, **characterized in that** the counter-plate (20) is rigidly connected to the base part (18) or is designed as part of the base part (18).

## Revendications

1. Prothèse comprenant une tige de prothèse (1) qui est réalisée pour recevoir un moignon d'amputation et qui comporte à son extrémité distale un système de solidarisation (3) pour retenir un membre artificiel (9), dans laquelle le système de solidarisation comprend une partie de base surfacique (18) avec une bordure de forme annulaire et un moyen de solidarisation capable de rotation autour d'un axe longitudinal dressé perpendiculairement à la partie de base surfacique (18), susceptible d'être positionné par rapport à la partie de base (18) et servant à la solidarisation du membre artificiel (9), le moyen de solidarisation pouvant être immobilisé dans une position angulaire réglée par rapport à la partie de base (18) au moyen d'un système de fixation et la bordure de forme annulaire entoure de manière à la guider une périphérie de forme circulaire du moyen de solidarisation, **caractérisée en ce que** la partie de base (18) est formée de manière intégrée solidairement en rotation avec sa bordure de forme annulaire dans l'extrémité distale de la tige de prothèse (1), et **en ce que** le système de fixation est réalisé pour l'immobilisation de la position angulaire en raison d'une force axiale exercée au moyen d'au moins une liaison à vis (23, 26) susceptible d'être actionnée depuis la face inférieure distale de la tige de prothèse (1).

2. Prothèse selon la revendication 1, **caractérisée en ce que** le moyen de solidarisation comprend une plaque de solidarisation (19) susceptible d'être tirée avec une force axiale contre une première butée axiale (21) de la partie de base (18), et une contre-plaque (20) permettant la force axiale et soutenue axialement sur la partie de base (18).

3. Prothèse selon la revendication 2, **caractérisée en ce que** la partie de base (18) est réalisée sous forme d'une bague avec une paroi intérieure comportant la première butée axiale (21) pour le moyen de solidarisation.

4. Prothèse selon la revendication 3, **caractérisée en ce que** la paroi intérieure comporte des butées axiales (21, 22) dans les deux directions axiales, et **en ce que** la contre-plaque (20) s'appuie sur l'une des butées axiales (21, 22) en direction opposée par rapport à la plaque de fixation (19).

5. Prothèse selon la revendication 3 ou 4, **caractérisée en ce que** la contre-plaque (20) comporte une périphérie extérieure ronde, et **en ce qu'**une bordure de forme annulaire de la partie de base (18) entoure la contre-plaque (20) en la guidant.

6. Prothèse selon la revendication 2 ou 3, **caractérisée en ce que** la contre-plaque (20) est fermement reliée à la partie de base (18) ou réalisée en tant que partie de la partie de base (18).
